# EUROPEAN PATENT SPECIFICATION

(11) **EP 3 881 704 B1**
(45) Date of publication and mention of the grant of the patent: **16.11.2022**
(21) Application number: 21158155.8
(22) Date of filing: 19.02.2021
(51) Int. Cl.: A41D 19/00, A41D 19/015, A61B 42/10, B29D 99/00

(54) **RUBBER GLOVE**
GUMMIHANDSCHUH
GANT EN CAOUTCHOUC

(30) Priority: 18.03.2020 JP 2020047370
(43) Date of publication of application: 22.09.2021
(73) Proprietor: Sumitomo Rubber Industries, Ltd., Kobe-shi, Hyogo-ken 651-0072 (JP)
(72) Inventor: TAKAI, Atsushi, Kobe-shi, Hyogo 651-0072 (JP); OTA, Erika, Kobe-shi, Hyogo 651-0072 (JP); NAGAKI, Masahiro, Kobe-shi, Hyogo 651-0072 (JP); PEL SZU, Lean, 08000 Sungai Petani, Kedah (MY); PERUMAL, Punitha A/P, 08000 Sungai Petani, Kedah (MY)
(74) Representative: Manitz Finsterwald Patent- und Rechtsanwaltspartnerschaft mbB

(56) References cited:
- CN-A- 108 602 214

## Description

### BACKGROUND OF THE INVENTION

### 1. Field of the Invention

The present invention relates to a rubber glove.

### 2. Description of the Related Art

Rubber gloves formed of rubber films are used in a large number of applications in general households and in various fields such as industrial production, research, medical treatment, and sports from the viewpoints of dexterity, low cost, and the like. Among them, for industrial work rubber gloves, excellent chemical resistance is required in order to protect hands from chemicals.

In the field where industrial work rubber gloves are used, chemicals with a wide range of polarity are used. Gloves made of acrylonitrile-butadiene rubber are commonly used as rubber gloves resistant to non-polar solvents such as hexane and heptane. However, the gloves made of acrylonitrile-butadiene rubber do not have sufficient resistance to polar solvents such as acetone and alcohol. On the other hand, gloves made of polyethylene, polyvinyl alcohol, and butyl rubber have been developed as rubber gloves having excellent resistance to polar solvents. However, the gloves made of polyethylene do not fit to the hands well and therefore have poor workability. The gloves made of polyvinyl alcohol have poor water resistance. The gloves made of butyl rubber are cumbersome to manufacture. Furthermore, these gloves do not have sufficient resistance to non-polar solvents.

On the other hand, rubber gloves with a multilayer structure including a rubber layer having excellent resistance to a polar solvent and a rubber layer having excellent resistance to a non-polar solvent have been proposed (for example, see Patent Literatures 1 and 2). Specifically, Patent Literature 1 proposes a rubber glove having a multilayer structure of a chloroprene rubber layer and an acrylonitrile-butadiene rubber layer. Patent Literature 2 proposes a rubber glove having a multilayer structure of a chloroprene rubber layer and a mixed rubber layer of acrylonitrile-butadiene rubber and chloroprene rubber. Patent Literature 3 shows a rubber glove comprising: an layer composed of a crosslinked product of a rubber composition containing an acrylonitrile-butadiene rubber and an outermost layer composed of a crosslinked product of a rubber composition (2) comprising an acrylonitrile-butadiene rubber and a chloroprene rubber, wherein the two layer and the outer layer are adjacent to each other.

### CITATION LIST

### Patent Literature

Patent Literature 1: WO-A-2017/041134
Patent Literature 2: JP-A-2010-133068
Patent Literature 3: CN 108 602 214 A

### SUMMARY OF THE INVENTION

### Technical Problem

However, as a result of intensive studies by the present inventors, it has been found that the adhesion between layers is poor and delamination may occur in the rubber gloves of the above-mentioned prior arts. In addition, it has been found that the rubber gloves of the above-mentioned prior arts have a problem that the resistance to polar solvents such as ketones tends to be low. Further, it has been found that in the rubber gloves of the above-mentioned prior arts, the dexterity may be insufficient due to the stiffness.

In view of the above circumstances, it is an object of the present invention to provide a rubber glove having a multilayer structure, which has high adhesion between layers, excellent resistance to ketones, and excellent dexterity.

### Solution to Problem

The present invention is a rubber glove including: an inner layer composed of a crosslinked product of a rubber composition (1) containing acrylonitrile-butadiene rubber; and an outer layer composed of a crosslinked product of a rubber composition (2) containing acrylonitrile-butadiene rubber and chloroprene rubber, wherein the inner layer and the outer layer are adjacent to each other, and two or more types of chloroprene rubber having different crystallization rates are used as the chloroprene rubber.

### Advantageous Effects of Invention

According to the present invention, it is possible to provide a rubber glove having a multilayer structure, which has high adhesion between layers, excellent resistance to ketones, and excellent dexterity.

### DESCRIPTION OF THE PREFERRED EMBODIMENTS

The rubber glove of the present invention includes an inner layer composed of a crosslinked product of a rubber composition (1) containing acrylonitrile-butadiene rubber and an outer layer composed of a crosslinked product of a rubber composition (2) containing acrylonitrile-butadiene rubber and chloroprene rubber. Here, the inner layer and the outer layer are adjacent to each other. As the chloroprene rubber, two or more types of chloroprene rubber having different crystallization rates are used.

### [Inner layer]

The inner layer is composed of a crosslinked product of a rubber composition (1) containing an acrylonitrile-butadiene rubber. Thus, the inner layer is a crosslinked rubber layer. The rubber glove of the present invention has an inner layer using an acrylonitrile-butadiene rubber, whereby high resistance to a non-polar solvent is obtained.

As the acrylonitrile-butadiene rubber, known acrylonitrile-butadiene rubber can be used, and it is also available as a commercial product. A functional group such as a carboxyl group may be introduced into the acrylonitrile-butadiene rubber.

Examples of commercially available acrylonitrile-butadiene rubber, Nipol 1042, 1052J, DN202, DN212, DN219, DN225, DN3335, DN3350, DN3390, 1043, DN302, DN302H, DN306, DN2950, DN401, DN401L, DN401LL, DN406, DN407 manufactured by Zeon Corporation; N238H, N232SH, N237H, N230S, N232S, N237, N233, N230SL, N231L, N230SV, N239SV, N241H, 242S, N250S, N260S, N250SL manufactured by JSR Corporation; and the like. Acrylonitrile-butadiene rubber may also be included in latex, examples of which include Nipol 1562, 1571H, 1571C2, 1571CL, 1577K, LX511A, LX513, manufactured by Zeon Corporation; KNL830, KNL833, KNL835, KNL850, KNL870 manufactured by Kumho Petrochemical Co., Ltd.; SYNTHOMER X6617 manufactured by SYNTHOMER plc; Lutex N117 manufactured by LG Chem, Ltd., and the like.

The rubber composition (1) may contain a rubber component other than acrylonitrile-butadiene rubber within a range that does not significantly impair the effects of the present invention. When the rubber component other than the acrylonitrile-butadiene rubber is contained, the content thereof is preferably 10% by mass or less in the rubber component of the rubber composition (1).

The rubber composition (1) typically contains sulfur as a curing agent. The content of the sulfur is not particularly limited. The content of the sulfur is typically 0.5 parts by mass or more and 6.0 parts by mass or less, preferably 1.0 parts by mass or more and 3.0 parts by mass or less, more preferably 1.0 parts by mass or more and 2.0 parts by mass or less, with respect to 100 parts by mass of the rubber component in the rubber composition (1).

The rubber composition (1) may contain components other than acrylonitrile-butadiene rubber and a curing agent. Examples of such components include cure activators, cure accelerators, dispersion stabilizers, pigments, anti-aging agents, plasticizers, fillers, pH adjustment agents, impurities derived from the production of acrylonitrile-butadiene rubber (e.g., emulsifiers, dispersants, catalysts, catalyst activators, chain transfer agents, polymerization inhibitors, which are added during polymerization), and the like.

Examples of the cure activator include metal oxides such as zinc oxide and magnesium oxide, and among these, zinc oxide is preferable. The content of the cure activator is typically 1.0 parts by mass or more and 6.0 parts by mass or less, preferably 1.5 parts by mass or more and 4.0 parts by mass or less, with respect to 100 parts by mass of the rubber component in the rubber composition (1).

As the cure accelerator, a known chemical used in the rubber layer of a rubber glove may be used. Examples thereof include thiourea-based cure accelerators, guanidine-based cure accelerators, dithiocarbamate-based cure accelerators, thiuram-based cure accelerators, and the like. The cure accelerators can be used either alone or in combination with two or more. As the cure accelerator, a dithiocarbamate-based cure accelerator is preferable.

Examples of the thiourea-based cure accelerator include ethylene thiourea (EU), diethyl thiourea (DEU), dibutyl thiourea (DBTU), diphenyl thiourea (DPTU), diorthotolyl thiourea (DOTU), trimethyl thiourea (TMU), and the like. Among these, DPTU is preferable.

Examples of the guanidine-based cure accelerator include 1,3-diphenylguanidine (DPG), 1,2,3-triphenylguanidine (TPG), 1,3-di-o-tolylguanidine (DOTG), 1-(o-tolyl)biguanide (OTBG), and the like. Among them, DPG is preferable.

Examples of the dithiocarbamate-based cure accelerator include zinc dimethyldithiocarbamate (PZ), zinc diethyldithiocarbamate (EZ), zinc dibutyldithiocarbamate (BZ), zinc N-ethyl-N-phenyldithiocarbamate (PX), nickel dibutyldithiocarbamate (NBC), and the like. Among these, BZ is preferable.

Examples of the thiuram-based cure accelerator include tetramethylthiuram disulfide (TT), tetraethylthiuram disulfide (TET), tetrabutylthiuram disulfide (TBT), and the like.

The content of the cure accelerator in the rubber composition (1) is not particularly limited. The content of the cure accelerator is typically 0.5 parts by mass or more and 5 parts by mass or less with respect to 100 parts by mass of the rubber component in the rubber composition (1).

As the dispersion stabilizer, a known chemical used in the rubber layer of a rubber glove may be used. Examples thereof include ammonia casein, potassium hydroxide, surfactants (particularly nonionic surfactants), and the like. The content of the dispersion stabilizer is not particularly limited, but is typically 0.05 parts by mass or more and 2.5 parts by mass or less with respect to 100 parts by mass of the rubber component in the rubber composition (1).

As the filler, a known material used in a rubber layer of a rubber glove may be used. Examples thereof include calcium carbonate, magnesium carbonate, carbon black, silica, alumina, titanium oxide, talc, mica, kaolin clay, hard clay, and the like. The content of the filler is not particularly limited, but is typically 1 part by mass or more and 20 parts by mass or less, preferably 5 parts by mass or more and 15 parts by mass or less, with respect to 100 parts by mass of the rubber component in the rubber composition (1).

The method of allowing the rubber composition (1) to be a crosslinked product is not particularly limited, and a known curing method for forming a rubber layer of a rubber glove can be applied.

### [Outer layer]

The outer layer is composed of a crosslink product of a rubber composition (2) containing acrylonitrile-butadiene rubber and chloroprene rubber. Thus, the outer layer is a crosslinked rubber layer. The rubber glove of the present invention has an outer layer using a chloroprene-based rubber, whereby high resistance to a polar solvent can be obtained. In addition, since the outer layer contains not only chloroprene-based rubber but also acrylonitrile-butadiene-based rubber, the outer layer also has resistance to non-polar solvents, and the adhesion with the inner layer can be made stronger.

Examples of the acrylonitrile-butadiene rubber include the same acrylonitrile-butadiene rubber used for the inner layer. The acrylonitrile-butadiene rubber contained in the outer layer may be the same as or different from the acrylonitrile-butadiene rubber contained in the inner layer.

As the chloroprene rubber, a homopolymer of chloroprene and a copolymer of chloroprene and a monomer copolymerizable therewith can be used. Examples of the monomers copolymerizable with chloroprene include ethylene; styrene; acrylonitrile; (meth)acrylic acid; (meth)acrylamide; (meth)acrylic esters such as methyl (meth)acrylate, ethyl (meth)acrylate, butyl (meth)acrylate, 2-ethylhexyl (meth)acrylate, 2-methoxyethyl (meth)acrylate, 2-hydroxyethyl (meth)acrylate; butadienes such as butadiene, 2,3-dichloro-1,3-butadiene, 1-chloro-1,3-butadiene; isoprene; and the like. The content ratio of the monomer units copolymerizable with chloroprene to all the monomer units constituting the copolymer is typically 50 mol% or less, preferably 30 mol% or less, more preferably 10 mol% or less. The chloroprene rubber is commercially available, and a commercially available chloroprene rubber may be used. The chloroprene rubber may be included in the latex.

However, in the present invention, two or more types of chloroprene rubber having different crystallization rates are used as the chloroprene rubber. In other words, in the present invention, chloroprene rubber (A) having a low crystallization rate and chloroprene rubber (B) having a high crystallization rate are used at least for the chloroprene rubber. It should be noted that the "low" and "high" in the crystallization rate is based on comparison between chloroprene rubber (A) and chloroprene rubber (B).

The chloroprene rubbers having various crystallization rates are known. When the crystallization rates are classified into, for example, seven levels (seven ranges) of "extremely slow", "very slow", "slow", "intermediate", "fast", "very fast", and "extremely fast", it is preferable that the crystallization rate of the chloroprene rubber (A) is in the "intermediate range to the fast range" and the crystallization rate of the chloroprene rubber (B) is in the "very fast range".

As an index of the crystallization rate, for dry chloroprene-based rubbers stored at -10°C, the hardness increase time (R) required for the surface hardness measured in accordance with JIS K6301:1996 to rise by 10 points from the initial hardness at time zero (0) is mentioned. The shorter the hardness increase time (R), the higher the crystallization rate.

Representative examples of chloroprene rubber having a crystallization rate in the range from the intermediate range to the fast range include "Neoprene 671A" manufactured by DuPont. This "Neoprene 671A" manufactured by DuPont is also available as "Showa Denko Chloroprene 671A" manufactured by Showa Denko K. K. Therefore, as the chloroprene rubber (A), a rubber is used whose hardness increase time (R_{A}) is preferably in the range of hardness increase time (R_{NA}) of Neoprene 671A ±20 hours, more preferably in the range of hardness increase time (R_{NA}) of Neoprene 671A ±15 hours, and further preferably in the range of hardness increase time (R_{NA}) of Neoprene 671A ±10 hours. It should be noted that Neoprene 671A is a latex, and R_{NA} of Neoprene 671A is a measure for dried matter thereof.

Typical examples of chloroprene rubber having a crystallization rate in the very fast range include "Neoprene 572" manufactured by DuPont. The "Neoprene 572" manufactured by DuPont is also available as "Showa Denko Chloroprene 572" manufactured by Showa Denko K. K. Therefore, as the chloroprene rubber (B), a rubber is used whose hardness increase time (R_{B}) is preferably in the range of hardness increase time (R_{NB}) of Neoprene 572 ±15 hours, more preferably in the range of hardness increase time (R_{NB}) of Neoprene 572 ±10, and further preferably in the range of hardness increase time (R_{NB}) of Neoprene 572 ±5. It should be noted that Neoprene 572 is a latex, and R_{NB} of Neoprene 572 is a measure for dried matter thereof. In addition, the hardness increase time (R_{A}) > the hardness increase time (R_{B}) is satisfied.

The crystallization rate of the chloroprene rubber can be controlled by changing the polymerization temperature at the time of manufacturing the chloroprene rubber. When the chloroprene rubber is a copolymer containing the copolymerizable monomer unit, the crystallization rate can be controlled by changing the type of the copolymerizable monomer unit and the content ratio thereof.

The mass ratio of the chloroprene rubber (A) to the chloroprene rubber (B) is not particularly limited. The mass ratio (chloroprene rubber (A):chloroprene rubber (B)) is, for example, 15:85 to 85:15, preferably 25:75 to 75:25, more preferably 30:70 to 70:30.

The mass ratio of the chloroprene rubber and the mass ratio of the acrylonitrile-butadiene rubber to the total mass of the chloroprene rubber and the acrylonitrile-butadiene rubber in the rubber composition (2) are not particularly limited. Since the balance between mechanical strength and chemical resistance is particularly excellent, preferably, the mass ratio of chloroprene rubber (the total of two or more chloroprene rubbers having different crystallization rates) is 75 mass % or more and 97.5 mass % or less, and the mass ratio of acrylonitrile-butadiene rubber is 2.5 mass % or more and 25 mass % or less, to the total mass. More preferably, the mass ratio of the chloroprene rubber is 85% by mass or more and 95% by mass or less, and the mass ratio of the acrylonitrile-butadiene rubber is 5% by mass or more and 15% by mass or less, to the total mass.

The rubber composition (2) may contain rubber components other than acrylonitrile-butadiene rubber and chloroprene rubber within a range that does not significantly impair the effects of the present invention.

The rubber composition (2) typically contains a curing agent. The curing agent contained in the rubber composition (2) is preferably zinc oxide. The content of the curing agent is not particularly limited. In the case where zinc oxide is used as the curing agent, when the content of zinc oxide is too small, the chemical resistance and mechanical strength tend to be low. Therefore, the content of zinc oxide is preferably 0.3 parts by mass or more, more preferably 0.5 parts by mass or more, still more preferably 1 part by mass or more, and most preferably 2.5 parts by mass or more, with respect to 100 parts by mass of the rubber component contained in the rubber composition (2). On the other hand, also when the content of zinc oxide is too large, the chemical resistance and mechanical strength tend to be low. Therefore, the content of zinc oxide is preferably 10 parts by mass or less, more preferably 8.5 parts by mass or less, still more preferably 6 parts by mass or less, and most preferably 5.5 parts by mass or less, with respect to 100 parts by mass of the rubber component contained in the rubber composition (2). In particular, in the range where the content of zinc oxide is 1 part by mass or more and 6 parts by mass or less, chemical resistance becomes remarkably high.

The rubber composition (2) may contain components other than acrylonitrile-butadiene rubber, chloroprene rubber, and curing agent. Examples thereof include cure accelerators, dispersion stabilizers, pigments, anti-aging agents, plasticizers, fillers, pH adjustment agents, impurities derived from the manufacture of chloroprene-based rubbers and acrylonitrile-butadiene-based rubbers (e.g., emulsifiers, dispersing agents, catalysts, catalyst activators, chain transfer agents, polymerization inhibitors, which are added during polymerization), and the like.

Examples of the cure accelerator included in the rubber composition (2) are the same as the cure accelerator included in the rubber composition (1). As the cure accelerator, a combination of a thiourea-based chemical and a guanidine-based chemical is preferable. The content of the cure accelerator in the rubber composition (2) is not particularly limited. The content of the cure accelerator is typically 0.5 parts by mass or more and 10 parts by mass or less, preferably 2.5 parts by mass or more and 8 parts by mass or less, with respect to 100 parts by mass of the rubber component in the rubber composition (2).

As the dispersion stabilizer included in the rubber composition (2), the same dispersion stabilizer as that included in the rubber composition (1) is exemplified. The content thereof is also the same as that of the rubber composition (1).

Examples of the filler included in the rubber composition (2) include the same filler as that included in the rubber composition (1). The content thereof is also the same as that of the rubber composition (1).

The method of allowing the rubber composition (2) to be a crosslinked product is not particularly limited, and a known curing method for forming a rubber layer of a rubber glove can be applied.

### [Rubber gloves]

In the rubber glove of the present invention, the inner layer and the outer layer are adjacent to each other, and thus, the inner layer and the outer layer are in close contact with each other. In this specification, the "inner layer" means a layer on the side where the hand of the user is touching.

The total thickness of the inner layer and the outer layer is not particularly limited. Dexterity tends to be better in the case where the total thickness is thinner, but chemical resistance and mechanical strength tend to be worse. Therefore, the total thickness is preferably 0.30 mm or more, more preferably 0.35 mm or more, and still more preferably 0.40 mm or more. On the other hand, if the total thickness is large, the chemical resistance is increased, while the dexterity tends to be worse. Therefore, the total thickness is preferably 0.70 mm or less, more preferably 0.60 mm or less, and still more preferably 0.50 mm or less.

In addition, when the ratio of the thickness of the outer layer to the total thickness is small, the resistance to the polar solvent tends to be worse. Therefore, the ratio of the thickness of the outer layer to the total thickness is preferably 0.5 or more, more preferably 0.6 or more. On the other hand, if the ratio of the thickness of the outer layer to the total thickness is large, the resistance to the non-polar solvent tends to be worse. Therefore, the ratio of the thickness of the outer layer to the total thickness is preferably 0.9 or less, more preferably 0.8 or less.

In particular, when the total thickness is within the range of 0.40 mm or more and 0.50 mm or less, and the ratio of the thickness of the outer layer to the total thickness is within the range of 0.6 or more and 0.8 or less, the balance among the dexterity, the resistance to the polar solvent, and the resistance to the non-polar solvent is particularly excellent.

The rubber glove of the present invention may further have layers other than the inner layer and the outer layer described above.

The method for producing the rubber glove of the present invention is not particularly limited, and the rubber glove of the present invention can be produced according to a known method, for example, a dipping method. Specifically, for example, first, a latex compound (latex compound for inner layer) containing the component of the rubber composition (1) for forming the inner layer is prepared. A latex compound (latex compound for outer layer) containing the components of the rubber composition (2) for forming the outer layer is prepared. On the other hand, a mold made of, for example, ceramic corresponding to the three-dimensional shape of a rubber glove is prepared, and the surface thereof is treated with a coagulant, mainly calcium nitrate aqueous solution.

After the mold is immersed in the latex compound for outer layer and withdrawn out, the mold with the latex compound is heated and dried. This forms a glove-shaped outer layer. Next, this is immersed in the latex compound for inner layer and withdrawn out. This is then heated for drying and cure. By reverse stripping this from the mold, a rubber glove having an outer layer formed on the inner layer is obtained.

The rubber glove of the present invention has an advantage that the inner layer and the outer layer are difficult to peel off. The rubber gloves of the present invention are also highly resistant to both polar and non-polar solvents, particularly to ketones, especially acetone. Further, the rubber glove of the present invention is excellent in dexterity. Therefore, it can be suitably used for rubber gloves for industrial work used in, for example, factories, laboratories, and the like.

### EXAMPLES

Explanations will now be given of examples relating to the present invention, but it is not intended that the present invention is limited to these examples.

### Examples and Comparative examples

Acrylonitrile-butadiene latex ("KNL870" manufactured by KUMHO Petrochemical Co., Ltd.) was used to prepare mixtures containing the components listed in Table 1. The mixture was maturated for 2 days to prepare latex compound A for forming an inner layer.

**Table 1**

| Components | | Amount (parts by mass) |
|---|---|---|
| NBR | Rubber component of KNL 870 | 100 |
| Dispersion stabilizers | Ammonia casein | 0.3 |
| | KOH | 0.5 |
| | Nonionic surfactant | 0.5 |
| Vulcanizing components | Sulfur | 1.5 |
| | Zinc oxide | 3.0 |
| | BZ | 0.5 |

| | | |
|---|---|---|
| ^{∗} Abbreviations in the table are as follows. NBR: acrylonitrile-butadiene rubber BZ: Zinc dibutyldithiocarbamate | | |

On the other hand, acrylonitrile-butadiene latex " KNL870" manufactured by Petrochemical Co., Ltd., chloroprene latex "Neoprene 671A", and chloroprene latex "Neoprene 572" were used to prepare mixtures containing the components shown in Tables 2 to 4. Each mixture was maturated for 2 days to prepare latex compound B for forming an outer layer. The numerical values for each component in the table indicate the amount of blending (parts by mass).

Next, a ceramic mold with a hand shape corresponding to the three-dimensional shape of the glove was prepared. The mold was warmed at 60°C for 20 minutes, then immersed in a 25% calcium nitrate aqueous solution, withdrawn out and heated at 60°C for 1 minute to dry.

The mold was immersed in the latex compound B for forming an outer layer for 20 seconds and then withdrawn out. This was placed in a dryer and dried at 100°C for 5 minutes. Subsequently, this was immersed in the latex compound A for forming an inner layer and then withdrawn out. This was placed in a dryer, heated at 100°C for 30 minutes, and further heated at 130°C for 30 minutes. By reverse stripping this from the mold, a rubber glove was obtained. The thicknesses of the inner layer and the outer layer of the rubber glove were adjusted by changing the dwell time.

### [Mechanical Properties Measurement: Tensile Strength and Tensile Elongation Evaluation]

A dumbbell No. 6 type test piece was punched out from the rubber gloves of each of the above Examples and Comparative Examples, and the tensile strength and tensile elongation were measured according to JIS K6251:2010 using the test piece. Measurement results are shown in Tables 2 to 4.

### [Chemical Resistance Evaluation]

As resistance to polar solvents, the breakthrough times (minutes) were determined using acetone and methanol as solvents according to EN 374:2016. As resistance to non-polar solvents, the breakthrough times (minutes) were determined using toluene and heptane as solvents in accordance with EN 374:2016. Tables 2 to 4 shows the test results.

### [Dexterity Evaluation]

Ten monitors (people) wore the rubber gloves of each of the above Examples and Comparative Examples, and the dexterity (degree of less stiffness) was evaluated according to the following criteria. Evaluation results are given in Tables 2 to 4.
A: No stiffness at all (soft) and excellent dexterity
B: Little stiffness and good dexterity
C: Slight stiffness but fair dexterity
D: Strong stiffness and bad dexterity

### [Delamination Evaluation]

Ten rubber gloves of Examples and Comparative Examples were prepared. These were examined for the presence or absence of delamination, and the occurrence rate of delamination was calculated and evaluated based on the following criteria. Evaluation results are given in Tables 2 to 4.
A: 0% to 10% incidence of delamination
B: 20% to 50% incidence of delamination
C: 60% to100% incidence of delamination

**Table 2**

| | | Examples | | | | | | |
|---|---|---|---|---|---|---|---|---|
| | | 1 | 2 | 3 | 4 | 5 | 6 | 7 |
| CR | Rubber component of Neoprene 671A | 42 | 60 | 32 | 44 | 42 | 42 | 42 |
| | Rubber component of Neoprene 572 | 50 | 32 | 60 | 52 | 50 | 50 | 50 |
| NBR | Rubber component of KNL 870 | 8 | 8 | 8 | 4 | 8 | 8 | 8 |
| Dispersion stabilizers | Ammonia casein | 0.3 | 0.3 | 0.3 | 0.3 | 0.3 | 0.3 | 0.3 |
| | KOH | 0.2 | 0.2 | 0.2 | 0.2 | 0.2 | 0.2 | 0.2 |
| | Nonionic surfactant | 0.2 | 0.2 | 0.2 | 0.2 | 0.2 | 0.2 | 0.2 |
| Curing components | Sulfur | | | | | | | |
| | Zinc oxide | 8 | 8 | 8 | 8 | 5 | 3 | 0.5 |
| | DPTU | 3 | 3 | 3 | 3 | 3 | 3 | 3 |
| | DPG | 3 | 3 | 3 | 3 | 3 | 3 | 3 |
| | BZ | | | | | | | |
| Filler | Calcium carbonate | 10 | 10 | 10 | 10 | 10 | 10 | 10 |
| | Total thickness of inner and outer layers (mm) | 0.40 | 0.40 | 0.40 | 0.40 | 0.40 | 0.40 | 0.40 |
| | Thickness ratio (outer layer : inner layer) | 7:3 | 7:3 | 7:3 | 7:3 | 7:3 | 7:3 | 7:3 |
| Mechanical properties | Tensile strength (N/cm) | 35 | 36 | 35 | 37 | 40 | 39 | 36 |
| | Tensile elongation (%) | 375 | 380 | 365 | 355 | 390 | 400 | 410 |
| Chemical resistance: Breakthrough time (min) | Acetone | 13 | 13 | 13 | 15 | 18 | 21 | 10 |
| | Methanol | 120 | 120 | 120 | 120 | 120 | 120 | 120 |
| | Toluene | 10 | 10 | 10 | 11 | 12 | 12 | 10 |
| | Heptane | >480 | >480 | >480 | >480 | >480 | >480 | >480 |
| Dexterity | | B | B | B | B | B | B | B |
| Delamination evaluation | | A | A | A | A | A | A | A |

**Table 3**

| | | Examples | | | | | | | | |
|---|---|---|---|---|---|---|---|---|---|---|
| | | 8 | 9 | 10 | 11 | 12 | 13 | 14 | 15 | 16 |
| CR | Rubber component of Neoprene 671A | 42 | 42 | 42 | 42 | 42 | 42 | 42 | 42 | 42 |
| | Rubber component of Neoprene 572 | 50 | 50 | 50 | 50 | 50 | 50 | 50 | 50 | 50 |
| NBR | Rubber component of KNL 870 | 8 | 8 | 8 | 8 | 8 | 8 | 8 | 8 | 8 |
| Dispersion stabilizers | Ammonia casein | 0.3 | 0.3 | 0.3 | 0.3 | 0.3 | 0.3 | 0.3 | 0.3 | 0.3 |
| | KOH | 0.2 | 0.2 | 0.2 | 0.2 | 0.2 | 0.2 | 0.2 | 0.2 | 0.2 |
| | Nonionic surfactant | 0.2 | 0.2 | 0.2 | 0.2 | 0.2 | 0.2 | 0.2 | 0.2 | 0.2 |
| Curing components | Sulfur | | | | | | | | | |
| | Zinc oxide | 8 | 8 | 8 | 8 | 8 | 8 | 8 | 8 | 8 |
| | DPTU | 3 | 3 | 3 | 3 | 3 | 3 | 3 | 3 | 3 |
| | DPG | 3 | 3 | 3 | 3 | 3 | 3 | 3 | 3 | 3 |
| | BZ | | | | | | | | | |
| Filler | Calcium carbonate | 10 | 10 | 10 | 10 | 10 | 10 | 10 | 10 | 10 |
| | Total thickness of inner and outer layers (mm) | 0.35 | 0.42 | 0.48 | 0.55 | 0.60 | 0.40 | 0.40 | 0.40 | 0.40 |
| | Thickness ratio (outer layer : inner layer) | 7:3 | 7:3 | 7:3 | 7:3 | 7:3 | 9:1 | 8:2 | 6:4 | 5:5 |
| Mechanical properties | Tensile strength (N/cm) | 30 | 37 | 42 | 48 | 52 | 33 | 34 | 36 | 37 |
| | Tensile elongation (%) | 370 | 375 | 370 | 370 | 360 | 350 | 365 | 400 | 410 |
| Chemical resistance: Breakthrough time (min) | Acetone | 9 | 14 | 19 | 23 | 26 | 18 | 15 | 8.5 | 8 |
| | Methanol | 120 | 120 | 120 | 240 | 240 | 120 | 120 | 120 | 120 |
| | Toluene | 9 | 11 | 12 | 14 | 16 | 9 | 9 | 10 | 12 |
| | Heptane | 240 | >480 | >480 | >480 | >480 | 240 | 240 | >480 | >480 |
| Dexterity | | A | B | B | C | C | B | B | B | B |
| Delamination evaluation | | A | A | A | A | A | A | A | A | A |

**Table 4**

| | | Comparative examples | | | | | | | |
|---|---|---|---|---|---|---|---|---|---|
| | | 1 | 2 | 3 | 4 | 5 | 6 | 7 | 8 |
| CR | Rubber component of Neoprene 671A | 92 | 80 | 80 | 100 | | | | |
| | Rubber component of Neoprene 572 | | | | | 92 | 92 | | |
| NBR | Rubber component of KNL 870 | 8 | 20 | 20 | | 8 | 8 | 100 | 100 |
| Dispersion stabilizers | Ammonia casein | 0.3 | 0.3 | 0.3 | 0.3 | 0.3 | 0.3 | 0.3 | 0.3 |
| | KOH | 0.2 | 0.2 | 0.2 | 0.2 | 0.2 | 0.2 | 0.2 | 0.2 |
| | Nonionic surfactant | 0.2 | 0.2 | 0.2 | 0.2 | 0.2 | 0.2 | 0.2 | 0.2 |
| Curing components | Sulfur | | | | | | | 2 | 2 |
| | Zinc oxide | 8 | 8 | 8 | 8 | 8 | 8 | 3 | 3 |
| | DPTU | 3 | 3 | 3 | 3 | 3 | 3 | 3 | 3 |
| | DPG | 3 | 3 | 3 | 3 | 3 | 3 | 3 | 3 |
| | BZ | | | | | | | 2 | 2 |
| Filler | Calcium carbonate | 10 | 10 | 10 | 10 | 10 | 10 | 10 | 10 |
| | Total thickness of inner and outer layers (mm) | 0.40 | 0.40 | 0.55 | 0.40 | 0.40 | 0.35 | 0.40 | 0.55 |
| | Thickness ratio (outer layer : inner layer) | 7:3 | 7:3 | 7:3 | 7:3 | 7:3 | 7:3 | 7:3 | 7:3 |
| Mechanical properties | Tensile strength (N/cm) | 35 | 36 | 49 | 35 | 33 | 28 | 20 | 27 |
| | Tensile elongation (%) | 385 | 365 | 360 | 390 | 355 | 360 | 550 | 545 |
| Chemical resistance: Breakthrough time (min) | Acetone | 13 | 6.5 | 7.5 | 13 | 14 | 9 | 4.5 | 6.5 |
| | Methanol | 120 | 120 | 240 | 120 | 120 | 60 | 120 | 240 |
| | Toluene | 10 | 10 | 9 | 9 | 11 | 9 | 12 | 14 |
| | Heptane | >480 | >480 | >480 | >480 | >480 | 240 | >480 | >480 |
| Dexterity | | B | B | D | B | D | D | B | D |
| Delamination evaluation | | C | A | A | C | A | A | A | A |

| | | | | | | | | | |
|---|---|---|---|---|---|---|---|---|---|
| ^{∗} Abbreviations in the table are as follows. CR: Chloroprene rubber NBR: acrylonitrile-butadiene rubber DPTU: diphenylthiourea DPG: diphenylguanidine BZ: Zinc dibutyldithiocarbamate | | | | | | | | | |

From the above results, it can be seen that when the rubber glove includes an inner layer of acrylonitrile-butadiene rubber and an outer layer of a mixed rubber of acrylonitrile-butadiene rubber and chloroprene rubber, and two or more types of chloroprene rubber having different crystallization rates are used as the chloroprene rubber, the rubber glove is excellent in all of chemical resistance including ketones, dexterity, and suppression of delamination. It can also be seen that in this case, it has mechanical properties suitable for rubber gloves. Therefore, according to the rubber glove of the present invention, the adhesion between the layers is high, the resistance to ketones is excellent, and the dexterity is excellent.

## Claims

1. A rubber glove comprising:
an inner layer composed of a crosslinked product of a rubber composition (1) containing an acrylonitrile-butadiene rubber, and an outer layer composed of a crosslinked product of a rubber composition (2) comprising an acrylonitrile-butadiene rubber and a chloroprene rubber, wherein the inner layer and the outer layer are adjacent to each other, and two or more types of chloroprene rubber having different crystallization rates are used as the chloroprene rubber.

2. The rubber glove according to claim 1, wherein a total thickness of the inner layer and the outer layer is 0.40mm or more and 0.50mm or less, and a ratio of a thickness of the outer layer to the total thickness is 0.6 or more and 0.8 or less.

3. The rubber glove according to claim 1 or 2, wherein the rubber composition (2) contains zinc oxide in an amount of 1.0 parts by mass or more and 6.0 parts by mass or less with respect to 100 parts by mass of the rubber component.

4. The rubber glove according to any one of claims 1 to 3, wherein a mass ratio of the chloroprene rubber is 85% by mass or more and 95% by mass or less, and a mass ratio of the acrylonitrile-butadiene rubber is 5% by mass or more and 15% by mass or less, with respect to the total mass of the acrylonitrile-butadiene rubber and the chloroprene rubber in the rubber composition (2).

## Patentansprüche

1. Gummihandschuh, welcher umfasst:
eine innere Schicht, die aus einem vernetzten Produkt einer Kautschukzusammensetzung (1) aufgebaut ist, die einen Acrylnitril-Butadien-Kautschuk enthält, und
eine äußere Schicht, die aus einem vernetzten Produkt einer Kautschukzusammensetzung (2) aufgebaut ist, die einen Acrylnitril-Butadien-Kautschuk und einen Chloropren-Kautschuk umfasst, wobei die innere Schicht und die äußere Schicht zueinander benachbart sind, und zwei oder mehr Arten von Chloropren-Kautschuk, die unterschiedliche Kristallisationsgeschwindigkeiten aufweisen, als der Chloropren-Kautschuk verwendet werden.

2. Gummihandschuh nach Anspruch 1, wobei eine Gesamtdicke der inneren Schicht und der äußeren Schicht 0,40 mm oder mehr und 0,50 mm oder weniger beträgt, und ein Verhältnis einer Dicke der äußeren Schicht zu der Gesamtdicke 0,6 oder mehr und 0,8 oder weniger beträgt.

3. Gummihandschuh nach Anspruch 1 oder 2, wobei die Kautschukzusammensetzung (2) Zinkoxid in einer Menge von 1,0 Massenteilen oder mehr und 6,0 Massenteilen oder weniger enthält, bezogen auf 100 Massenteile der Kautschukkomponente.

4. Gummihandschuh nach einem der Ansprüche 1 bis 3, wobei ein Massenanteil des Chloropren-Kautschuks 85 Massenprozent oder mehr und 95 Massenprozent oder weniger beträgt, und ein Massenanteil des Acrylnitril-Butadien-Kautschuks 5 Massenprozent oder mehr und 15 Massenprozent oder weniger beträgt, bezogen auf die Gesamtmasse des Acrylnitril-Butadien-Kautschuks und des Chloropren-Kautschuks in der Kautschukzusammensetzung (2).

## Revendications

1. Gant en caoutchouc comprenant :
une couche intérieure composée d'un produit réticulé en composition de caoutchouc (1) contenant un caoutchouc acrylonitrile butadiène, et
une couche extérieure composée d'un produit réticulé en composition de caoutchouc (2) comprenant un caoutchouc acrylonitrile butadiène et un caoutchouc chloroprène, dans lequel
la couche intérieure et la couche extérieure sont adjacentes l'une à l'autre, et
deux ou plusieurs types de caoutchouc chloroprène ayant des taux de cristallisation différents sont utilisés comme caoutchouc chloroprène.

2. Gant en caoutchouc selon la revendication 1, dans lequel une épaisseur totale de la couche intérieure et de la couche extérieure est de 0,40 mm ou plus et de 0,50 mm ou moins, et un rapport d'une épaisseur de la couche extérieure sur l'épaisseur totale est de 0,6 ou plus et de 0,8 ou moins.

3. Gant en caoutchouc selon la revendication 1 ou 2, dans lequel la composition de caoutchouc (2) contient de l'oxyde de zinc dans une quantité de 1,0 part en masse ou plus et de 6,0 parts en masse ou moins par rapport à 100 parts en masse du composant en caoutchouc.

4. Gant en caoutchouc selon l'une quelconque des revendications 1 à 3, dans lequel un rapport de masse du caoutchouc chloroprène est de 85 % en masse ou plus et de 95 % en masse ou moins, et un rapport de masse du caoutchouc acrylonitrile butadiène est de 5 % en masse ou plus et de 15 % en masse ou moins, par rapport à la masse totale du caoutchouc acrylonitrile butadiène et du caoutchouc chloroprène dans la composition de caoutchouc (2).
